# EUROPÄISCHE PATENTSCHRIFT

(11) **EP 3 744 812 B1**
(45) Veröffentlichungstag und Bekanntmachung des Hinweises auf die Patenterteilung: **12.01.2022**
(21) Anmeldenummer: 19176668.2
(22) Anmeldetag: 27.05.2019
(51) Int. Cl.: C10B 53/07, C25B 1/00, C08G 18/00, C07C 263/00, C07C 263/10

(54) **VERFAHREN ZUR VERWERTUNG VON POLYURETHAN MATERIAL ABFALL ZUR HERSTELLUNG VON CHEMIKALISCHEN ROHSTOFFEN FÜR DIE HERSTELLUNG VON ISOCYANATEN UND POLYURETHANEN**
METHOD FOR THE RECYCLING OF POLYURETHANE MATERIAL WASTE FOR THE PRODUCTION OF CHEMICAL RAW MATERIALS FOR THE PRODUCTION OF ISOCYANATES AND POLYURETHANES
PROCÉDÉ D'UTILISATION DE DÉCHETS DU MATÉRIAU DE TYPE POLYURÉTHANE PERMETTANT LA FABRICATION DE MATIÈRES PREMIÈRES CHIMIQUES POUR LA FABRICATION D'ISOCYANATES ET DE POLYURÉTHANES

(43) Veröffentlichungstag der Anmeldung: 02.12.2020
(73) Patentinhaber: Covestro Deutschland AG, 51373 Leverkusen (DE)
(72) Erfinder: BULAN, Andreas, 40764 Langenfeld (DE); WEBER, Rainer Dr., 51519 Odenthal (DE)
(74) Vertreter: Levpat

(56) Entgegenhaltungen:
- US-A- 4 243 560
- US-A1- 2007 276 154
- US-A1- 2018 194 632

## Beschreibung

Die Erfindung betrifft ein Verfahren zur Verwertung von Polyurethan-Material Abfall zur Herstellung von chemikalischen Rohstoffen für die Herstellung von Isocyanaten und anschließend Polyurethanen, bei dem ausgehend von Polyurethan-Material Abfall durch Pyrolyse, Kohlendioxid und Kohlenwasserstoffe und gegebenenfalls Kohlenmonoxid und Wasserstoff erzeugt werden, das Kohlendioxid durch Elektrolyse zu Kohlenmonoxid und ggf. Wasserstoff umgesetzt wird, , erhaltenes Kohlenmonoxid über Phosgen zu Isocyanat umgesetzt wird und das Isocyanat zu neuem Polyurethan-Material weiterverarbeitet werden kann.

Die Erfindung betrifft besonders ein Verfahren zur emissionsarmen Herstellung von Isocyanaten und Polyolen unter Einsatz der elektrochemischen Umsetzung von Kohlendioxid zu Kohlenmonoxid und ggf. Wasserstoff und Herstellung von Sauerstoff sowie der Nutzung des Sauerstoffs zur Verbrennung von Polyurethan enthaltenen Materialien zu Kohlendioxid und ggf. Verbrennung von Pyrolyserückständen erhalten aus Polyurethan enthaltenen Materialien und Nutzung des jeweilig erhaltenen Kohlendioxids als Rohstoff für die elektrochemische CO₂ Reduktion. Das erzeugte Kohlenmonoxid wird zur Herstellung von Isocyanaten und gegebenenfalls zur Herstellung von Methanol als Vorprodukt für Polyalkylenoxide verwendet

Das erhaltene Kohlenmonoxid und ggf. erzeugtes CO₂ wird dabei anschließend wahlweise weiter mit Wasserstoff zu Methanol und dieses weiter durch den Methanol to Olefine (MTO) Prozess bzw. speziell Methanol to Propen (MTP) Prozess zur Hauptkomponente Propen umgesetzt. Das Propen wird zu Propylenoxid und dieses dann zu Polyethern umgesetzt. Ein weiterer Teil des Kohlenmonoxid wird mit Chlor zu Phosgen und dieses mit Aminen zu Isocyanaten umgesetzt. Aus den Isocyanaten und den Polyethern können erneut wieder Polyurethan-Materialien hergestellt werden. Somit ist ein großer Teil der Wertschöpfungskette geschlossen. Bei Einsatz von CO₂ und regenerativer Energie für die Elektrolyse kann in nachhaltiger Weise Polyurethan Material hergestellt werden.

Weiter kann durch Integration einer Wasserelektrolyse zusätzlich benötigter Wasserstoff zur Hydrierung der Nitro-Verbindungen zu Aminen hergestellt werden, die mit dem Phosgen zu Isocyanaten umgewandelt werden können. Sowohl bei der elektrochemischen CO₂ Reduktion als auch bei der Wasserelektrolyse wird das Koppelprodukt Sauerstoff jeweils an der Anode erzeugt. Dieser Sauerstoff kann für die Verbrennung der Polyurethan-enthaltenden Abfall-Materialien und des Pyrolyserückstands eingesetzt werden, wodurch bei der Verbrennung ein hochkonzentrierter CO₂ Abgasstrom erhalten wird und die CO₂ Rückgewinnung hierdurch deutlich wirtschaftlicher wird, als bei der Verbrennung des Polyurethan-Material enthaltenen Abfalls mit Luft.

Polyurethane, nachfolgend auch PU genannt, sind Kunststoffe, die aus der Polyadditionsreaktion von Dialkoholen beziehungsweise Polyolen mit Polyisocyanaten entstehen. Diole und Diisocyanate führen zu linearen Polyurethanen, vernetzte Polyurethane können durch Umsetzung von Triisocyanat-Diisocyanat-Gemischen mit Triol-Diol-Gemischen hergestellt werden. Die Eigenschaften von PU können in einem weiten Rahmen variiert werden. Je nach Vernetzungsgrad und/oder eingesetzter Isocyanat- oder OH-Komponente erhält man Duroplaste, Thermoplaste oder Elastomere. Polyurethane werden jedoch auch als Formmassen zum Formpressen, als Gießharze (Isocyanat-Harze), als (textile) elastische Faserstoffe, Polyurethanlacke und als Polyurethanklebstoffe verwendet. Aus Polyurethan lassen sich auch sehr einfach Schaumstoffe herstellen.

Weiche PU-Schaumstoffe werden für sehr viele Zwecke verwendet, vor allem als Polstermaterial z. B. für Möbel und Autositze, als Matratzenschaum, als Teppichrückenmaterial, zur Textilkaschierung, als Reinigungsschwamm oder als Filtermaterial.

PU-Hartschäume werden vor allem zur Wärmedämmung z. B. in Gebäuden, Kühlgeräten, Wärme- und Kältespeichern sowie einigen Rohrsystemen (Kunststoffmantelverbundrohr, flexible Verbundrohre) eingesetzt.

Weitere, relativ neue Anwendungsgebiete für PU-Schäume gibt es im Fahrzeugbau wie Lenkrad, Armauflage, Softbeschichtung von Handgriffen, Innenraumverkleidung, Armaturenbrett, Schalldämmung, Klapperschutz, Abdichtungen, Transparentbeschichtung von Holzdekoren.

Nach dem Ablauf der Nutzungsphase der PU-Material enthaltenen Produkte werden diese üblicherweise entsorgt, d.h. auf Mülldeponien gelagert oder in Müllverbrennungsanlagen verbrannt. Eine stoffliche Nutzung konnte bisher noch nicht wirtschaftlich erfolgreich durchgeführt werden, d.h. dass es bisher nicht gelang, aus den PU-Materialien die eingesetzten Polyole bzw. Isocyanate in wirtschaftlicher Ausbeute wieder zu gewinnen.

Ein Ansatz zum stofflichen Recycling ist die Glykolyse, bei der die Urethangruppe mit Glykol zu Carbamat und einem Polyol umgesetzt wird.

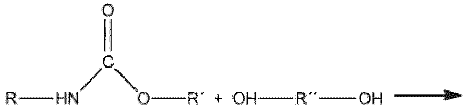

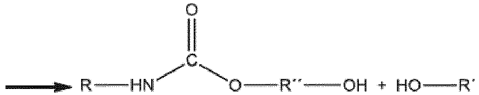

Weiterhin kann die Urethangruppe mit einem Amin zu Urea und einem Polyol umgesetzt werden.

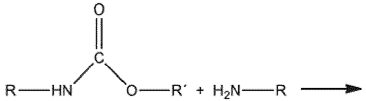

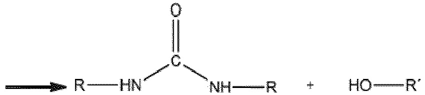

Ein aus dem Stand der Technik bekanntes Verfahren ist offenbart in dem Dokument US 4 243 560 A.

Aufgabe der vorliegenden Erfindung war es, ein nachhaltiges alternatives Verfahren für die Isocyanatherstellung und letztlich auch für die Polyurethan-Herstellung unter Einbeziehung von Recyclingprozessen und Schließung der Wertschöpfungsketten zu finden. Bisher werden wesentliche Komponenten zur Polyurethanherstellung wie das Kohlenmonoxid, Wasserstoff und Propen oder auch Ethen bzw. deren Oxide zur Herstellung von Polyolen aus fossilen Energieträgern hergestellt. So wird konventionell Kohlenmonoxid und Wasserstoff aus Erdgas bzw. aus Kohle mittels Reformingprozessen, und Propen und Ethen aus Erdölfraktionen gewonnen.

Eine Aufgabe der Erfindung ist es also den stofflichen Einsatz von fossilen Rohstoffen und gegebenenfalls auch den energetischen Einsatz von fossilen Rohstoffen für die Isocyanatproduktion zu vermindern. Hiermit soll die Kohlendioxid-Bilanz (Carbon Footprint) der PU-Produktion weiter verbessert werden.

Gegenstand der Erfindung ist ein Verfahren zur Verwertung von Polyurethan Material enthaltendem Abfall zur Herstellung von chemikalischen Rohstoffen für die Herstellung von Isocyanaten und Polyurethanen durch
a) Pyrolyse des Polyurethan Materials bei erhöhter Temperatur, gegebenenfalls in Gegenwart von Katalysator, unter Erhalt von gegebenenfalls Kohlendioxid, gegebenenfalls Kohlenmonoxid, gegebenenfalls Wasserstoff, einem Gemisch von aliphatischen und aromatischen niedermolekularen Kohlenwasserstoffen und stickstoffhaltigen Kohlenwasserstoffen und einem Rückstand von höher molekularen Kohlenwasserstoffen,
b) gegebenenfalls Raffination des in Schritt a) erhaltenen Gemisches von niedermolekularen Kohlenwasserstoffen unter Erhalt von einem Gemisch von gasförmigen und flüssigen Kohlenwasserstoffen und einem Gemisch von Kohlendioxid, Kohlenmonoxid und weiteren gasförmigen niedermolekularen Kohlenwasserstoffverbindungen, und Trennung der erhaltenen Gemische und,
c) Verbrennung des in Schritt a) erhaltenen Rückstands und gegebenenfalls von weiterem Polyurethan Materialabfall mit sauerstoffhaltigem Gas, insbesondere mit reinem Sauerstoff, unter Erhalt von Kohlendioxid enthaltendem Gas,
d) Reinigung des aus Schritt c) und gegebenenfalls aus Schritt a) erhaltenen Kohlendioxids von Nebenbestandteilen, insbesondere von Stickoxiden, Schwefelverbindungen, Staub, Wasser, Sauerstoff und HCl wahlweise mittels Adsorption, Membranverfahren oder Gaswäsche, oder katalytischer Behandlung, unter Erhalt eines gereinigten Kohlendioxids,
e) Elektrolyse des in Schritt d) erhaltenen gereinigten Kohlendioxids, insbesondere elektrochemische Umsetzung des Kohlendioxids an einer Gasdiffusionselektrode, unter Erhalt eines Gemischs mindestens von Kohlenmonoxid, nicht umgesetztem Kohlendioxid und gegebenenfalls Wasserstoff,
f) Abtrennung des nicht umgesetzten Kohlendioxids von dem in Schritt e) erhaltenen Gemisch unter Erhalt eines Gemischs von mindestens Kohlenmonoxid und gegebenenfalls Wasserstoff und Rückführung des nicht umgesetzten Kohlendioxids in die Elektrolyse,
g) optional Abtrennung des gegebenenfalls erhaltenen Wasserstoffs aus dem in Schritt f) erhaltenen Gemisch von Kohlenmonoxid und gegebenenfalls Wasserstoff,
h) Umsetzung des aus Schritt g) oder f) erhaltenen Kohlenmonoxids mit Chlor zu Phosgen in einem Prozess zur Herstellung von Isocyanat,
i) gegebenenfalls Umsetzung des aus Schritt h) erhaltenen Isocyanats mit Polyether und gegebenenfalls zusätzlich mit Polyester zu einem fertigen Polyurethan Material.

Bevorzugt wird der Sauerstoff für die Verbrennung in Schritt c) aus einer Wasserelektrolyse erhalten.

Durch Einsatz von Strom aus nachhaltiger Energieerzeugung (Windkraft, Wasserkraft, Sonnenenergie) wird die CO₂-Emission bei dem Gesamtverfahren weiter herabgesetzt. In einem bevorzugten neuen Verfahren wird der bei der Wasserelektrolyse gebildete Wasserstoff wahlweise bei der optionalen Raffination und/oder bei einer Hydrierung von Nitroaromaten verwendet, wobei die bei der Hydrierung von Nitroaromaten erhaltenen Amine in der Isocyanat Herstellung eingesetzt werden können. Der gegebenenfalls in Schritt g) des neuen Verfahrens erhaltene abgetrennte Wasserstoff wird bevorzugt bei der Hydrierung von Nitroaromaten eingesetzt. Hiermit werden Amine als Vorprodukte des Isocyanats zugänglich.

Der Stoffkreislauf wird in einer besonders bevorzugten Ausführung des erfindungsgemäßen Verfahren weiter dadurch geschlossen, dass das Polyurethan Material nach seiner Nutzung zu Polyurethan Abfallmaterial recykliert wird und der isolierte Polyurethan Abfall als Einsatzmaterial in Schritt a) des Gesamtverfahrens eingesetzt wird.

Unter weiteren gasförmigen niedermolekularen Kohlenwasserstoffverbindungen werden hier in der Raffination nach Schritt b) insbesondere gegebenenfalls stickstoffhaltige C₁ bis C₄-Kohlenwasserstoffe verstanden.

Beim Recycling von PU-Material nach dem Ende der Nutzungsdauer werden übliche Trennverfahren zur Abtrennung von Verbundstoffen im Abfall angewandt. So wird das PU-Material automatisiert oder händisch grob abgetrennt, dann mechanisch zerkleinert und ggf. weiter getrennt. Das erhaltene PU-Material dient als Rohstoff für die Verbrennung oder die Pyrolyse.

Im Falle der Verbrennung in Schritt c) wird das PU-Material beispielsweise mit reinem Sauerstoff O₂, welches als Nebenprodukt der CO₂-Elektrolyse oder der optionalen Wasserelektrolyse, an der Anode entwickelt wird, umgesetzt. Die bei der Verbrennung in Schritt c) anfallende Reaktionswärme kann zur Herstellung von Dampf und/oder von elektrischem Strom eingesetzt werden. Besonders kann die Wärme zum Betrieb der Pyrolyse in Schritt a) eingesetzt werden und der erzeugte elektrische Strom in der Elektrolyse in Schritt e). Hierdurch wird die Effizienz des neuen Gesamtverfahrens weiter verbessert.

Das aus der Verbrennung in Schritt c) stammende CO₂ fällt in hochkonzentrierter Form an und wird vor der weiteren Nutzung einer Reinigung in Schritt d) zugeführt. Hierbei werden die Nebenprodukte der Verbrennung, z.B. Schwefelverbindungen wie SO₂, Stickstoffverbindungen wie NOx sowie restliche Organika als auch Staub und andere entstandene Verbindungen, die aus denen im PU-Material vorhandenen Komponenten entstanden sind, abgetrennt.

Die Verbrennung des PU-Materials mit reinem Sauerstoff gemäß Schritt c) kann z.B. gemäß dem als Oxyfuel-Verfahren bekannten Verfahren in einer Atmosphäre aus reinem Sauerstoff und CO₂ (rezirkulierendem Rauchgas) erfolgen. Das dabei entstehende Rauchgas ist nicht mit dem in der Luft enthaltenen Stickstoff verdünnt und besteht im Wesentlichen aus CO₂ und Wasserdampf. Der Wasserdampf kann mit wenig Aufwand kondensiert werden, so dass ein hochkonzentrierter CO₂-Strom (Konzentration im Idealfall nahe 100 Prozent) entsteht. Das CO₂ kann dann gereinigt und weiterverarbeitet, ggf. auch verdichtet und gelagert werden.

Weiterhin kann ein Teil der Energie, die bei der Pyrolyse in Schritt a) oder bei der Verbrennung in Schritt c) des Polyurethanmaterials gewonnen wird in Dampf oder Elektrizität umgesetzt werden. Mit der gewonnenen Elektrizität kann die Elektrolyse in Schritt e) betrieben werden, womit ein noch effizienteres Verfahren mit geringem Verbrauch an elektrischer Energie entsteht.

Die Reinigung des CO₂ aus Verbrennungsgasen kann nach aus dem Stand der Technik grundsätzlich bekannten Verfahren erfolgen. Dies wird im folgenden beispielhaft beschrieben.

Zuerst erfolgt dabei z.B. eine Reinigung der Verbrennungsgase, deren Hauptbestandteil CO₂ ist. Der Aufbau einer Verbrennungsgasreinigung unterteilt sich in unterschiedliche Stufen. Besondere Aufgabe der Reinigung ist es, ein CO₂ ohne störende Nebenbestandteile für die nachfolgende, im weiteren beschriebene bevorzugte elektrochemische Reduktion von CO₂ an einer Gasdiffusionselektrode bereit zu stellen.

In der ersten Stufe wird Staub aus dem Verbrennungsgas entfernt. Dies kann mit Gewebefiltern oder mit einem Elektrofilter erfolgen. Danach können ggf. vorhandene saure Gas, wie Chlorwasserstoff, der sich aus im Abfall vorhandenen Chlor-Verbindungen bildet, entfernt werden. Hierbei werden z.B. Abgaswaschtürme eingesetzt. Das Verbrennungsgas wird hierbei auch abgekühlt und von weiteren Stäuben und ggf. Schwermetallen befreit. Weiterhin wird auch gebildetes Schwefeldioxid-Gas in einem Waschkreislauf abgeschieden und z.B. mit Kalkhydrat zu Gips umgesetzt. Die Entfernung von Stickstoffverbindungen aus den Verbrennungsgasen kann z.B. an katalysatorenthaltenen Zeolithen oder durch Zugabe von Harnstoff oder Ammoniak die Stickoxide wieder zu Stickstoff und Wasser umgewandelt werden. Um eine Entstehung von Ammoniumsalzen zu verhindern, die die Katalysatorporen verstopfen würden, erfolgt der Betrieb der Katalysatoren meist bei einer Temperatur von über 320 °C (beispielhaft grundsätzlich beschrieben bei: https://de.wikipedia.org/wiki/Rauchgasreinigung). Ebenso können die N₂-Verbindungen durch Wäsche mit Salpetersäure oder eine Wäsche mit Katalysatoren entfernt werden.

Die Trocknung und weitere Reinigung des CO₂ kann durch übliche bekannte Verfahren erfolgen. Trocknung z.B. durch eine Behandlung mit konz. Schwefelsäure.

In der letzten Reinigungsstufe werden Aktivkohlefilter eingesetzt, um noch im Verbrennungsgas enthaltene restliche Organika sowie letzte Reste von Metallen durch Aktivkohle zu entfernen. Hierzu kann z.B. staubförmige Aktivkohle in den Verbrennungsgasstrom bzw. Rauchgasstrom dosiert zugeführt und anschließend zusammen mit den angelagerten Schadstoffen auf den Gewebefilter wieder abgeschieden werden. Die verbrauchte Kohle wird ausgeschleust und der energetischen Verwertung zugeführt (grundsätzlich beschrieben in: https://www.ava-augsburg.de/umwelt/rauchgasreinigung/).

Nach den durchgeführten Reinigungsverfahren der Verbrennungsgase steht ein CO₂ zur Verfügung, welches in Schritt e) als Rohstoff eingesetzt werden kann.

Optional kann auch die CO₂ Abtrennung mittels Aminwäsche aus Gasströmen mit geringerer Konzentration an CO₂ erfolgen.

Das wie zuvor beschrieben recycelte und zerkleinerte PU-Material kann der Pyrolyse in Schritt a) zugeführt werden, wobei die Pyrolyse wahlweise mit oder ohne Katalysator durchgeführt werden kann.

Die bei der Pyrolyse entstehenden Fraktionen sind gasförmig, flüssig und fest, wobei die feste Phase meist vorwiegend aus pyrolytischem Kohlenstoff besteht. Die flüssigen langkettigen KohlenstoffVerbindungen, enthaltend Aromaten wie Toluol, Benzol, Xylol, werden bevorzugt einem Raffinationsprozess in Schritt c) zugeführt. Hier können die Verbindungen aufgetrennt oder in Raffinationsprozessen ggf. mit Wasserstoff, vorzugsweise Wasserstoff aus der Wasserelektrolyse weiter umgesetzt werden, so dass im Ergebnis auch Propen und Ethen (als Vorprodukte für Polyole, Polyether) erhalten werden können. Die langkettigen, flüssigen Kohlenwasserstoff-Verbindungen können abgetrennt und weiterverarbeitet werden. Auch die aromatischen Verbindungen wie Benzol oder Anilin oder, falls vorkommend auch Isocyanate, könnten in den entsprechenden Synthesen als Rohstoffe wieder eingesetzt werden.

Weiterhin kann die Pyrolyse in Schritt a) wahlweise insbesondere so betrieben werden, dass größere Mengen an Kohlenmonoxid und ggf. Wasserstoff erzeugt werden. Diese Gase können gemeinsam mit den kurzkettigen Kohlenwasserstoff-Verbindungen z.B. in der Raffination abgetrennt oder auch separat abgetrennt werden und dann einer Kohlenmonoxid-Wasserstoff-Trennung (7) zugeführt und verwendet werden.

Die bei der Pyrolyse in Schritt a) anfallenden festen Stoffe, bestehen meist aus Kohlenstoff. Diese feste Phase kann mit reinem Sauerstoff aus der CO₂-Elektrolyse oder aus der bevorzugt eingesetzten Wasserelektrolyse umgesetzt werden. Hierbei entsteht ebenfalls ein hoch konzentrierter Stoffstrom an CO₂, welcher einer Reinigung zugeführt wird.

Eine andere Möglichkeit zur Herstellung von hochreinem CO₂ ist die Absorption des CO₂ in Alkalilauge, zum Beispiel Kalilauge. Hierbei bildet sich Kaliumhydrogencarbonat, welches anschließend wieder zu CO₂ und Kalilauge thermisch zersetzt werden kann. Hierbei kann erzeugte Wärme aus der Pyrolyse oder Verbrennung eingesetzt werden.

Das gereinigte CO₂ wird bevorzugt dem Kathodenraum einer CO₂ -Elektrolyse gemäß Schritt e) zugeführt.

Hierbei kann die CO₂-Elektrolyse beispielsweise eine Hochtemperaturelektrolyse sein, die bei einer Temperatur von mehr als 600°C ggf. auch unter Zusatz von Wasser zur Herstellung von Synthesegas, betrieben wird. Hochtemperaturelektrolysen sind grundsätzlich bekannt und im Markt verfügbar z.B. von Haldor Topsoe, eCOs^{™} (https://www.topsoe.com/processes/carbon-monoxide/site-carbonmonoxide). Bei der Hochtemperaturelektrolyse entsteht an der Anode ebenfalls Sauerstoff. Nachteil der bekannten Hochtemperaturelektrolyse ist ihre schlechte Scale-up Fähigkeit, so dass bei größeren COMengen, z.B. mehr als 1 t/h CO, derzeit noch die Niedertemperaturelektrolyse zu bevorzugen ist.

Wird die CO₂ -Elektrolyse als Niedertemperaturelektrolyse betrieben, so erfolgt die Elektrolyse bei einer Temperatur unter 150°C.

Bei allen CO₂ -Elektrolysen wird das gereinigte CO₂ Gas dem Kathodenraum zugeführt.

Im Falle der Niedertemperaturelektrolyse wird CO₂ insbesondere an einer Gasdiffusionselektrode zu Kohlenmonoxid und ggf. Wasserstoff umgesetzt. Gleichzeitig kann an der Anode O₂ oder ggf. alternativ auch Chlor erzeugt werden.

Nach den bekannten Prinzipien kann bei der Niedertemperatur-Elektrolyse auch ein MEA (Membran-Electrode-Assembly) Konzept zum Einsatz kommen. Hierbei wird auf die Membran ein Katalysator aufgebracht. Eine davor gelagerte Gasdiffusionsschicht reguliert den Gas- und Flüssigkeitstransport. Dies kann sowohl auf der Anoden- als auch auf der Kathodenseite erfolgen. Weiterhin besteht die Möglichkeit eine Gasdiffusionselektrode in direktem Kontakt mit der Membran zu bringen.

Wird an der Anode Chlor erzeugt, so kann dieses Chlor der Phosgensynthese und somit der Isocyanat-Herstellung als weiterer Rohstoff zugeführt werden.

Im Kathodenraum wird z.B. eine Gasdiffusionselektrode installiert. Dies kann wie auch bei der Chlor/Alkalielektrolyse in zero-gap als auch in finite-gap Anordnung (COV-Patentanmeldung COV 101 186) erfolgen.

Dem Kathodenraum, in dem die Gasdiffusionselektrode betrieben wird, kann ein Überschuss an CO₂ zugeführt werden. Überschuss bedeutet, mehr CO₂ einzuleiten als für die stöchiometrische Umsetzung aufgrund des fließenden elektrischen Stromes notwendig wäre. Somit gelangt aus dem Kathodenraum eine Gasmischung bestehend aus nicht umgesetzten CO₂, CO und H₂. Die gesamte Gasmischung kann beispielsweise direkt einer Methanolsynthese zugeführt werden (Stoffstrom 23). Hier erfolgt die Umsetzung des CO₂ zu Methanol mit ggf. zusätzlich zugeführtem Wasserstoff sowie die Umsetzung von CO mit H₂ zu Methanol. Der zusätzlich benötigte Wasserstoff wird dabei gegebenenfalls aus einer oben beschriebenen Wasserelektrolyse bezogen. In einer bevorzugten Ausführung des neuen Verfahrens wird das in Schritt f) erhaltene Gemisch (6b) aus Kohlenmonoxid und gegebenenfalls Wasserstoff einer Methanolsynthese (11) zugeführt. Das Methanol 11a wird dann auch Vorprodukt der Polyetherherstellung.

Soll aus dem Gasgemisch, das dem Kathodenraum bei der CO₂-Elektrolyse entnommen wird, Kohlenmonoxid abgetrennt werden, so wird das überschüssige nicht umgesetzte CO₂ zunächst in Schritt f) durch eine Gasabtrennung (6), z.B. durch eine Aminwäsche entfernt und das Restgas aus CO und H₂ einer Gastrenneinheit (7) in Schritt g) zugeführt. Das in Schritt g) erhaltene CO wird dann der Isocyanatherstellung (10) zugeführt, bei der es mit Chlor, z.B. aus einem HCl Recyclingprozess (9), zu Phosgen und das Phosgen mit Aminen (8a) (insbesondere mit Diaminen) zu Isocyanat (insbesondere zu Diisocyanaten) in der Isocyanat-Herstellung (10) umgesetzt wird.

Der aus der Wasserelektrolyse 3 bzw. der Gastrennung (7) in Schritt g) erhaltene Wasserstoff kann entweder zur Hydrierung der Nitroaromaten zu den Aminen (8a) und damit der Herstellung der Isocyanate (10) oder der Methanol-Herstellung (11) zugeführt werden.

Bevorzugt ist daher eine Ausführung des neuen Verfahrens, bei der mindestens Teilströme des Kohlenmonoxids (22) und/oder des Wasserstoffs (21) aus der Gastrennung (7) einer Methanolsynthese (11) zugeführt werden.

Das bei der bevorzugt eingesetzten Methanolsynthese entstehende Methanol 11a wird in einer weiteren bevorzugten Ausführung des neuen Verfahrens einem Methanol to Olefin (MTO) Prozess (12) und die hieraus erhaltenen Alkene werden anschließend einem Prozess (13) bzw. (14) zur Herstellung von Alkylenoxiden, insbesondere von Propylenoxid (13 a) und/oder von Ethylenoxid (14 a) zugeführt.

Angeschlossen an diese bevorzugte Herstellung von Alkylenoxiden wird in einer besonders bevorzugten Ausführung des neuen Verfahrens das Propylenoxid (13 a) und/oder das Ethylenoxid (14 a) aus der Propylenoxidherstellung (13) bzw. aus der Ethylenoxidherstellung (14) einer Polyether Herstellung (15) zugeführt und der hieraus erhaltene Polyether (15 a) wird im Herstellungsprozess (16) zur Herstellung von neuem Polyurethan Material eingesetzt.

Hiermit wird auch das weitere für die Polyurethanherstellung benötigte Rohmaterial, der Polyether, in besonders ressourcenschonender Weise zugänglich.

Das gegebenenfalls bei der Raffination in Schritt b) erhaltene Gasgemisch (19 b) aus Kohlendioxid, Kohlenmonoxid und gasförmigen Kohlenwasserstoffen wird in einer bevorzugten Variante des neuen Verfahrens ebenfalls der Verbrennung (2) in Schritt c) zugeführt.

Alternativ wird das gegebenenfalls bei der Raffination in Schritt b) erhaltene Gasgemisch (19 b) aus Kohlendioxid, Kohlenmonoxid und gasförmigen Kohlenwasserstoffen in einer bevorzugten weiteren Variante des neuen Verfahrens in einer Gastrennung (30) in die Komponenten: Kohlendioxid (2b), Kohlenmonoxid (7c) und Kohlenwasserstoffe (30a) aufgetrennt und die aufgetrennten Komponenten (2b; 7c; 30a) werden einzeln weiterverwendet; insbesondere wird das abgetrennte Kohlendioxid (2b) der Reinigung (4) in Schritt d) zugeführt.

Das aus der vorgenannten optionalen Gastrennung 30 erhaltene Kohlenmonoxid (7c) wird in einer besonders bevorzugten Ausführung der Isocyanatherstellung (10), z.B. zur Aminherstellung wie oben beschrieben, zugeführt.

Das neue Verfahren kann auch bevorzugt so betrieben werden, dass ein Teil des Polyurethan Materials anstelle der Pyrolyse 1 in Schritt a) direkt der Verbrennung (2) in Schritt c) zugeführt wird.

Es ist auch eine Ausführung des neuen Verfahrens bevorzugt, bei der mindestens ein Teil des in Schritt e) aus der Elektrolyse (5) erhaltenen Gemisches (23) aus Kohlendioxid, Kohlenmonoxid und gegebenenfalls Wasserstoff direkt der zuvor beschriebenen Methanolsynthese (11) zugeführt wird.

Sauerstoff, der bei der Umsetzung durch Elektrolyse (5) von Kohlendioxid (4a) in Schritt e) gebildet wird, wird in einer bevorzugten Variante des neuen Verfahrens mindestens teilweise der Verbrennung (2) in Schritt c) zugeführt.

Die Gesamtstoffbilanz wird weiter verbessert, indem in einem bevorzugten neuen Verfahren ein Teil des bei der Abtrennung (6) in Schritt f) erhaltenen Kohlendioxids (6a) wahlweise in den Eingangsstrom (4a) der Elektrolyse (5) in Schritt e) und/oder der optionalen Methanolsynthese (11) zugeführt wird.

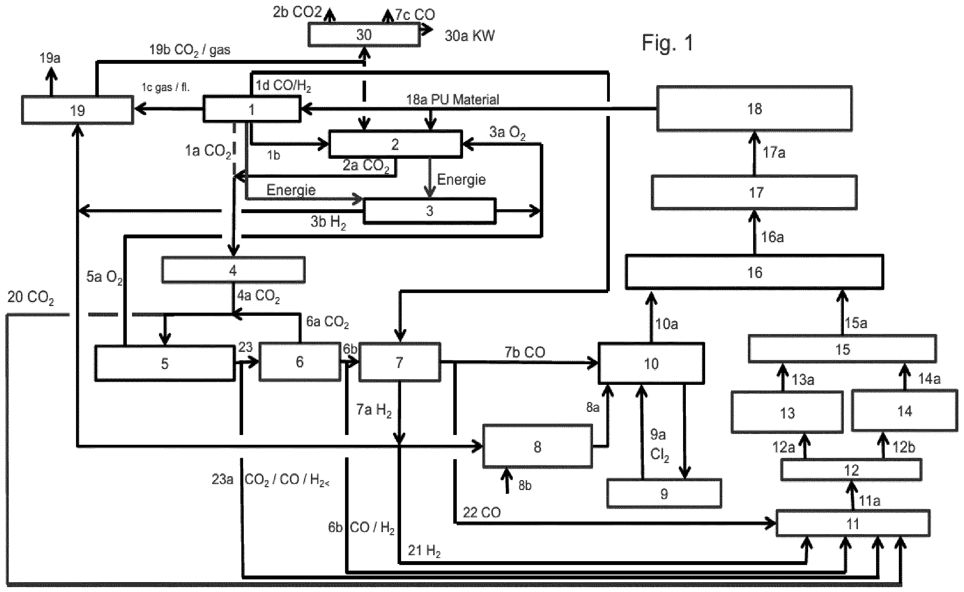

Der bei der Isocyanat-Herstellung anfallende Chlorwasserstoff (HCl) kann der Niedertemperatur CO₂-Elektrolyse (5) zugeführt (in Fig. 1 nicht gezeichnet) oder einer anderen HCl-Recyclingseinheit (9) wie z.B. einer HCl-Diaphragma oder HCl-Elektrolyse mit Gasdiffusionselektrode oder einer katalytischen Gasphasenoxidation zugeführt werden. Im Falle der HCl-Elektrolyse mit Gasdiffusionselektrode oder einer Gasphasenoxidation kann der benötigte O₂ aus der Niedertemperatur oder Hochtemperatur CO₂-Elektrolyse (5) und/oder der Wasserelektrolyse (3) bezogen werden (in Fig. 1 nicht gezeichnet).

Das in den bevorzugten Ausführungen des neuen Verfahrens hergestellte Methanol wird durch den grundsätzlich bekannten MTO (Methanol to Olefin) Prozess (12) beispielsweise zu Propen oder Ethen umgewandelt. Die dabei entstehenden Nebenprodukte können in die optionale Raffination (19) in Schritt b) geführt und somit einer sinnvollen weiteren Verwertung zugänglich werden. Aus dem Propen und Ethen des MTO Prozesses (12) können über die bekannt Verfahren Propylenoxid (13a) und Ethylenoxid (14a) hergestellt werden, aus denen z.B. die Polyether (15a) hergestellt werden können. Mit den Isocyanaten und den Polyethern und gegebenenfalls zusätzlich mit Polyestern können dann die im Markt benötigten PU-Materialien (16a) hergestellt werden. Die Polyurethane werden in verschiedenen Anwendungen genutzt (17). Nach dem Ende ihrer Nutzungszeit werden die Materialien einem Recycling (18) zugeführt und hier die PU-Materialien abgetrennt. Das abgetrennte Material wird dann wieder der Pyrolyse (1) und/oder der Verbrennung (2) zugeführt.

Hierdurch werden keine weiteren fossilen Rohstoffe für die Isocyanat-Herstellung benötigt und es kann in nachhaltiger Weise Polyurethan Material hergestellt werden.

Die Erfindung wird nachstehend anhand der Figuren beispielhaft näher erläutert.

Es zeigen:
- Fig. 1: Eine schematische Übersicht über das Gesamtverfahren einschließlich der PU Herstellung, Verwendung und Recyklierung

In Figur 1 haben die folgenden Bezugszeichen die jeweils rechtsstehende Bedeutung:
- 1: Pyrolyse Einheit
- 1a: CO₂ aus Pyrolyse (optional, ggf. werden die gasförmigen Komponenten einer CO₂-Abtrennung zugeführt (Kalilauge, Bildung KHCO₃ und dessen Zersetzung - nicht gezeichnet)
- 1b: Rückstand fest, aus der Pyrolyse
- 1c: Gasförmige und flüssigen Komponenten aus der Pyrolyse
- 1d: CO - Wasserstoff-Mischung aus der Pyrolyse
- 2: Verbrennung von PU-Material mit reinem Sauerstoff
- 2a: CO₂ Gasstrom aus der Verbrennung 2
- 3: optionale Wasserelektrolyse
- 3a: Sauerstoff aus der Wasserelektrolyse, Anodenraum
- 3b: Wasserstoff aus der Wasserelektrolyse, Kathodenraum
- 4: CO₂ Reinigung
- 4a: CO₂ Gasstrom gereinigt
- 5: CO₂ Elektrolyse
- 5a: O₂ aus der CO₂-Elektrolyse, Anodenraum
- 6: CO₂ Abtrennung aus CO, H₂, CO₂ Gasgemisch
- 6a: CO₂ Gasstrom aus CO₂ Abtrennung
- 6b: CO / H₂ abgetrennt aus CO₂-Abtennung
- 7: CO₂-H₂ Gastrennung
- 7a: Wasserstoff aus der Gastrennung CO-H2 7
- 7b: Kohlenmonoxid aus der Gastrennung CO-H2 7
- 7c: Kohlenmonoxid aus der Gastrennung 30
- 8: Hydrierung von Nitroaromaten
- 8a: Amine
- 8b: Nitroaromat
- 9: HCl-Recycling
- 9a: Chlor aus dem HCl Recycling
- 10: Isocyanat-Herstellung
- 10 a: Isocyanate
- 11: Methanol-Synthese
- 11a: Methanol
- 12: Methanol zu Olefin Prozess (MTO)
- 12a: Propen
- 12b: Ethen
- 13: Propylenoxid-Herstellung
- 13a: Propylenoxid
- 14: Ethylenoxid-Herstellung
- 14a: Ethylenoxid
- 15: Polyether-Herstellung
- 15a: Polyether
- 16: Polyurethan-Material Herstellung
- 16a: PU-Material
- 17: Markt - Nutzung der Polyurethan-Materialien bis zum Nutzungsende
- 17a: PU-Material benutzt
- 18: Recycling von benutztem Polyurethan-Material unter Isolierung der Polyurethan-Komponente
- 18a: Polyurethan-Material zur Pyrolyse bzw. Verbrennung
- 19: Raffination
- 19a: Produkte aus der Raffination
- 19b: CO₂ / CO und nicht abgetrennte gasförmige Verbindungen
- 20: CO₂ aus der CO₂ Reinigung 4 bzw. CO₂ Abtrennung 6 zur Methanol-Synthese 11
- 21: Wasserstoff aus CO-H₂ Gastrennung 7 oder der Wasserelektrolyse 3
- 22: CO aus der CO-H₂ Gastrennung 7 (ggf. aus der Pyrolyse, nicht gezeichnet in Fig.1)
- 23: CO, CO₂, H₂-Gasmischung aus der Niedertemperatur- oder Hochtemperatur-CO₂ Elektrolyse

- 30: Gastrennung für CO₂, CO, Kohlenwasserstoffe aus Raffination 19
- 30a: Kohlenwasserstoffe aus der Gastrennung 30

### Beispiel

Beispiel 1 Pyrolyse mit Katalysator, Verbrennung der gasförmigen Fraktion gem. Fig.1

Es wurde ein PU-Material mit einer Elementzusammensetzung von Kohlenstoff 66,5 Gew.-%, Wasserstoff 6,6 Gew.-%, Stickstoff 7,2 Gew.-% und Sauerstoff 18.8 Gew.-% eingesetzt und in einer katalytischen Pyrolyse 1 behandelt. Das PU-Material wurde zuvor in einer Schneidmühle kleingeschnitten, danach das Material heiß gepresst und erneut zerkleinert, so dass alle Partikel einen Durchmesser von weniger als 4 mm aufwiesen. Dieses Material 18a wurde mit einem Zeolith-basierten Katalysator HZSM-5 Gewichtsverhältnis 1:1 gemischt und einer Wirbelschicht zugeführt. In der Wirbelschicht befand sich bereits vorab eingefüllter Katalysators HZSM-5. Die Wirbelschicht wurde bei 600°C betrieben.

Aus der Pyrolyse 1 wurden 25,0 Gew.-% des zugeführten Materials an festem Rückstand 1b (vorwiegend Kohlenstoff) erhalten. Weiterhin wurden 37% der eingesetzten Masse als gasförmige Produkte und 35% als flüssige Materialien 1c entnommen. Die gasförmigen Verbindungen bestehend aus CO₂, CO, Methan, Ethen, Ethan, Propen, Propan wurden ohne Raffination 19 direkt der Verbrennung 2 mit reinem Sauerstoff zugeführt.

Es werden 2838 t/a PU-Material aus dem Recycling von PU-Isolationsmaterial aus Kühlschränken eingesetzt. Dabei werden 1516 t/a der Pyrolyse 1 und 1322 t/a der Verbrennung 2 zugeführt. Aus Pyrolyse 1 und Verbrennung 2 werden 5580 t/a CO₂ 1a; 2a nach Reinigung 4 des CO₂ der Elektrolyse 5 zugeführt, wobei aus der Elektrolyse 5 eine Gasmischung 23 von 3505 t/a CO und 135 t/a H₂ entnommen werden können. Weiterhin werden dem Anodenraum der Elektrolyse 3081 t/a O₂ 5a entnommen und der Verbrennung 2 des PU-Materials 18a bzw. des Rückstands 1b zugeführt.

Die Elektrolyse 5 wird dabei zweckmäßigerweise gemäß der europäischen Patentanmeldung Anmeldenummer 18195279.7, Beispiel 1 betrieben. Es werden 160 Elemente von je 2,5m² Elektrodenfläche (CO₂ GDE) benötigt, die zu einem Elektrolyseur zusammen geschaltet werden. Die Betriebszeit beträgt 8500 h je Jahr. Die Elektrolyse wird bei einer Zellspannung von 3,17 V mit einer Stromausbeute bzgl. CO von 68% betrieben. Es werden 32.722 MWh an regenerativ erzeugter Energie, insbesondere Windstrom, verbraucht.

Die gasförmigen Verbindungen 19b aus der Pyrolyse 1 werden der Verbrennung 2 zugeführt. Die festen Verbindungen 1b aus der Pyrolyse 1 werden ebenfalls einer Verbrennung 2 zugeführt.

Aus der flüssigen Fraktion der Pyrolyse 1 können 182 t/a Heterocyklen, 139 t/a Benzol/Toluol-Gemisch, 27 t/a Xylol und Naphthalin-Gemisch und 182 t/a Anilin mit einer zusätzlichen Raffination 19 gewonnen werden.

Das aus der Niedertemperaturelektrolyse 5 hergestellte Gasgemisch 23 wird einer Aminwäsche zugeführt und das nicht umgesetzte CO₂ aus dem Gemisch 23 abgetrennt und in die Elektrolyse 5 zurückgeführt. Das von CO₂ befreite Gas 6b bestehend aus CO und H₂ wird einer CO-H₂ Trennung in Form einer einer Cold-box 7 zugeführt, in der die Trennung von CO und H₂ erfolgt. Das CO 7b wird mit dem aus dem HCl-Recycling 9 stammende Cl₂ 9a zu Phosgen umgesetzt und dieses mit Anilin zu Isocyanat 10a umgesetzt.

Ein Teilstrom des Gasgemisches 23a aus der Elektrolyse 5 bestehend aus CO₂, CO und H2 wird nach Trocknung der Methanolsynthese zugeführt und der zur Umsetzung benötigte Wasserstoff 3b auch aus der Wasserelektrolyse 3 bezogen. Das Methanol 11a wird anschließend mittels MTO Prozess zu Propylen 12a und dem Nebenprodukt Ethylen 12b umgesetzt, welche weiter in den Stufen 13 bzw. 14 zu Propylenoxid 13a und Ethylenoxid 14a umgesetzt werden. Etwaig fehlende Mengen an Propylen/Ethylen werden aus anderen Herstellprozessen zugeführt. Aus den Alkyloxiden werden in der Polyetherherstellung 15 die für die PU-Material-Herstellung benötigen Polyole 15a hergestellt. Aus den Isocyanaten 10a und den Polyolen 14a wird neues PU-Material hergestellt 16a. Dieses kann nach Nutzung als benutzes PU-Material 17a dem Recycling 18 zur Herstellung von PU-Rohmaterial18a für die Verbrennung 1 / Pyrolyse 2 zugeführt werden, wodurch sich der Wertschöpfungskreis schließt.

## Patentansprüche

1. Verfahren zur Verwertung von Polyurethan Material enthaltendem Abfall (18 a) zur Herstellung von chemikalischen Rohstoffen für die Herstellung von Isocyanaten und Polyurethanen durch
a) Pyrolyse (1) des Polyurethan Materials (18 a) bei erhöhter Temperatur, gegebenenfalls in Gegenwart von Katalysator, unter Erhalt von gegebenenfalls Kohlendioxid (1a), gegebenenfalls Kohlenmonoxid, gegebenenfalls/ Wasserstoff (1d), einem Gemisch (1c) von aliphatischen und aromatischen niedermolekularen Kohlenwasserstoffen und stickstoffhaltigen Kohlenwasserstoffen und einem Rückstand (1b) von höher molekularen Kohlenstoffverbindungen,
b) gegebenenfalls Raffination (19) des in Schritt a) erhaltenen Gemisches (1c) von niedermolekularen Kohlenwasserstoffen unter Erhalt von einem Gemisch (19 a) von gasförmigen und flüssigen Kohlenwasserstoffen und einem Gemisch (19 b) von Kohlendioxid und Kohlenmonoxid, Wasserstoff und weiteren gasförmigen Kohlenwasserstoffverbindungen, und Trennung der erhaltenen Gemische (19 b) in einer Gastrennung 30
c) Verbrennung (2) des in Schritt a) erhaltenen Rückstands (1b) und gegebenenfalls von weiterem Polyurethan Materialabfall (18 a) mit sauerstoffhaltigem Gas (3a), insbesondere mit reinem Sauerstoff, unter Erhalt von Kohlendioxid enthaltendem Gas (2a),
d) Reinigung (4) des aus Schritt c) und gegebenenfalls aus Schritt a) erhaltenen Kohlendioxids (1a) und (2a) von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids (4a),
e) Elektrolyse (5) des in Schritt d) erhaltenen gereinigten Kohlendioxids (4a), insbesondere elektrochemische Umsetzung des Kohlendioxids (4a) an einer Gasdiffusionselektrode, unter Erhalt eines Gemisches (23) mindestens von Kohlenmonoxid, nicht umgesetztem Kohlendioxid und gegebenenfalls Wasserstoff,
f) Abtrennung (6) des nicht umgesetzten Kohlendioxids (6a) von dem in Schritt e) erhaltenen Gemisch (23) unter Erhalt eines Gemischs (6b) von mindestens Kohlenmonoxid und gegebenenfalls Wasserstoff und Rückführung des nicht umgesetzten Kohlendioxids (6a) in die Elektrolyse (5),
g) optional Abtrennung (7) des gegebenenfalls erhaltenen Wasserstoffs (7a) aus dem in Schritt f) erhaltenen Gemisch (6b) von Kohlenmonoxid und gegebenenfalls Wasserstoff,
h) Umsetzung des aus Schritt g) oder f) erhaltenen Kohlenmonoxids (7b) oder (6b) mit Chlor (9a) zu Phosgen in einem Prozess (10) zur Herstellung von Isocyanat (10 a),
i) gegebenenfalls Umsetzung (16) des aus Schritt h) erhaltenen Isocyanats (10 a) mit Polyethern (15 a) und gegebenenfalls zusätzlich mit Polyester zu einem fertigen Polyurethan Material (16 a).

2. Verfahren gemäß Anspruch 1, **dadurch gekennzeichnet, dass** der Sauerstoff (3a) für die Verbrennung (2) in Schritt c) aus einer Wasserelektrolyse (3) oder der CO₂ Elektrolyse (5) erhalten wird.

3. Verfahren gemäß Anspruch 2, **dadurch gekennzeichnet, dass** der bei der Wasserelektrolyse (3) gebildete Wasserstoff (3b) wahlweise bei der Raffination (19) und/oder bei einer Hydrierung (8) von Nitroaromaten verwendet wird, wobei die bei der Hydrierung (8) von Nitroaromaten erhaltenen Amine (8a) in der Isocyanat Herstellung (10) eingesetzt werden.

4. Verfahren gemäß einem der Ansprüche 1bis 3, **dadurch gekennzeichnet, dass** der gegebenenfalls in Schritt g) erhaltene abgetrennte Wasserstoff (7a) bei der Hydrierung (8) von Nitroaromaten eingesetzt wird.

5. Verfahren gemäß einem der Ansprüche 1bis 4, **dadurch gekennzeichnet, dass** das Polyurethan Material (16 a) nach seiner Nutzung (17) zu Polyurethan Abfallmaterial (17a) recykliert wird und der isolierte Polyurethan Abfall als Einsatzmaterial (18 a) in Schritt a) eingesetzt wird.

6. Verfahren gemäß einem der Ansprüche 1 bis 5, **dadurch gekennzeichnet, dass** das in Schritt e) und f) erhaltene Gemisch (6b) aus Kohlenmonoxid ggf. Kohlendioxid und gegebenenfalls Wasserstoff einer Methanolsynthese (11) zugeführt wird.

7. Verfahren gemäß einem der Ansprüche 1 bis 6, **dadurch gekennzeichnet, dass** mindestens Teilströme des Kohlenmonoxids (22) und/oder des Wasserstoffs (21) aus der Gastrennung (7) bzw. der Wasserelektrolyse (3) einer Methanolsynthese (11) zugeführt werden.

8. Verfahren gemäß einem der Ansprüche 6 und 7, **dadurch gekennzeichnet, dass** das Methanol (11 a) aus der Methanolsynthese (11) einem Methanol zu Olefin (MTO) Prozess (12) und die hieraus erhaltenen Alkene anschließend einem Prozess (13) bzw. (14) zur Herstellung von Alkylenoxiden, insbesondere von Propylenoxid (13 a) und/oder von Ethylenoxid (14 a) zugeführt werden.

9. Verfahren gemäß Anspruch 8, **dadurch gekennzeichnet, dass** das Propylenoxid (13 a) und/oder das Ethylenoxid (14 a) aus der Propylenoxidherstellung (13) bzw. aus der Ethylenoxidherstellung (14) einer Polyether Herstellung (15) zugeführt werden und der hieraus erhaltene Polyether (15 a) im Herstellungsprozess (16) zur Herstellung von neuem Polyurethan Material eingesetzt wird.

10. Verfahren gemäß einem der Ansprüche 1 bis 9, **dadurch gekennzeichnet, dass** das bei der Raffination (19) in Schritt b) erhaltene Gasgemisch (19 b) aus Kohlendioxid, Kohlenmonoxid und gasförmigen Kohlenwasserstoffen und ggf. Wasserstoff der Verbrennung (2) in Schritt c) zugeführt wird.

11. Verfahren gemäß einem der Ansprüche 1 bis 9, **dadurch gekennzeichnet, dass** das bei der Raffination (19) in Schritt b) erhaltene Gasgemisch (19 b) in einer Gastrennung (30) in die Komponenten: Kohlendioxid (2b), Kohlenmonoxid (7c) und Kohlenwasserstoffe (30a) aufgetrennt wird und die Komponenten (2b; 7c; 30a) einzeln weiterverwendet werden, insbesondere dass das Kohlendioxid (2b) der Reinigung (4) in Schritt d) zugeführt wird.

12. Verfahren gemäß Anspruch 11, **dadurch gekennzeichnet, dass** das Kohlenmonoxid (7c) aus der Gastrennung (30) der Isocyanatherstellung (10) zugeführt wird.

13. Verfahren gemäß einem der Ansprüche 1 bis 12, **dadurch gekennzeichnet, dass** ein Teil des Polyurethan Materials (18 a) direkt der Verbrennung (2) in Schritt c) zugeführt wird.

14. Verfahren gemäß einem der Ansprüche 1 bis 13, **dadurch gekennzeichnet, dass** mindestens ein Teil des in Schritt e) aus der Elektrolyse (5) erhaltenen Gemisches (23) aus Kohlendioxid, Kohlenmonoxid und gegebenenfalls Wasserstoff direkt der Methanolsynthese (11) zugeführt wird.

15. Verfahren gemäß einem der Ansprüche 1 bis 14, **dadurch gekennzeichnet, dass** bei der Elektrolyse (5) von Kohlendioxid (4a) in Schritt e) Sauerstoff (5a) gebildet wird, der mindestens teilweise der Verbrennung (2) in Schritt c) zugeführt wird.

16. Verfahren gemäß einem der Ansprüche 1 bis 15, **dadurch gekennzeichnet, dass** ein Teil des bei der Abtrennung (6) in Schritt f) erhaltenen Kohlendioxids (6a) wahlweise in den Eingangsstrom (4a) der Elektrolyse (5) in Schritt e) und/oder der optionalen Methanolsynthese (11) zugeführt wird.

## Claims

1. Method for recycling waste containing polyurethane material (18 a) for producing chemical feedstock for the production of isocyanates and polyurethanes by
a) pyrolysis (1) of the polyurethane material (18 a) at elevated temperature, optionally in the presence of a catalyst, to obtain a mixture (1c) of aliphatic and aromatic low molecular weight hydrocarbons and nitrogenous hydrocarbons, with or without carbon dioxide (1a), with or without carbon monoxide, with or without hydrogen (1d), and a residue (1b) of higher molecular weight carbon compounds,
b) optionally refining (19) the mixture (1c) of low molecular weight hydrocarbons obtained in step a) to obtain a mixture (19 a) of gaseous and liquid hydrocarbons and a mixture (19 b) of carbon dioxide and carbon monoxide, hydrogen and other gaseous hydrocarbon compounds, and separation of the resulting mixtures (19 b) in a gas separation 30
c) incinerating (2) the residue (1b) obtained in step a) and optionally further polyurethane material waste (18 a) with oxygen-containing gas (3a), in particular with pure oxygen, to obtain gas containing carbon dioxide (2a),
d) purifying (4) the carbon dioxide (1a) and (2a), obtained from step c) and possibly from step a), of secondary constituents, in particular nitrogen oxides, sulfur compounds, dust, water, oxygen and HCl, optionally by means of adsorption, gas scrubbing or catalytic treatment to obtain purified carbon dioxide (4a),
e) electrolysis (5) of the purified carbon dioxide (4a) obtained in step d), in particular electrochemical conversion of the carbon dioxide (4a) at a gas diffusion electrode, to obtain a mixture (23) of at least carbon monoxide, unconverted carbon dioxide and possibly hydrogen,
f) separating (6) the unconverted carbon dioxide (6a) from the mixture (23) obtained in step e) to obtain a mixture (6b) of at least carbon monoxide and possibly hydrogen and recycling the unconverted carbon dioxide (6a) to the electrolysis (5),
g) optionally separating (7) the hydrogen (7a) possibly obtained from the mixture (6b) of carbon monoxide and possibly hydrogen obtained in step f),
h) reacting the carbon monoxide (7b) or (6b) obtained from step g) or f) with chlorine (9a) to form phosgene in a process (10) for producing isocyanate (10 a),
i) optionally reacting (16) the isocyanate (10 a) obtained from step h) with polyethers (15 a) and optionally additionally with polyester to give a finished polyurethane material (16 a).

2. Method according to Claim 1, **characterized in that** the oxygen (3a) for the incineration (2) in step c) is obtained from water electrolysis (3) or the CO₂ electrolysis (5).

3. Method according to Claim 2, **characterized in that** the hydrogen (3b) formed in the water electrolysis (3) is optionally used in the refining (19) and/or in a hydrogenation (8) of nitroaromatics, wherein the amines (8a) obtained in the hydrogenation (8) of nitroaromatics are used in the isocyanate production (10).

4. Method according to any of Claims 1 to 3, **characterized in that** the separated hydrogen (7a) possibly obtained in step g) is used in the hydrogenation (8) of nitroaromatics.

5. Method according to any of Claims 1 to 4, **characterized in that** the polyurethane material (16 a) is recycled after its use (17) to form polyurethane waste material (17a) and the isolated polyurethane waste is used as feed material (18 a) in step a).

6. Method according to any of Claims 1 to 5, **characterized in that** the mixture (6b) of carbon monoxide, possibly carbon dioxide and possibly hydrogen obtained in step e) and f) is fed to a methanol synthesis (11).

7. Method according to any of Claims 1 to 6, **characterized in that** at least partial streams of the carbon monoxide (22) and/or of the hydrogen (21) from the gas separation (7) or the water electrolysis (3) are fed to a methanol synthesis (11) .

8. Method according to either of Claims 6 and 7, **characterized in that** the methanol (11 a) from the methanol synthesis (11) is fed to a methanol to olefin (MTO) process (12) and the alkenes obtained therefrom are then fed to a process (13) or (14) for producing alkylene oxides, especially propylene oxide (13 a) and/or ethylene oxide (14 a).

9. Method according to Claim 8, **characterized in that** the propylene oxide (13 a) and/or the ethylene oxide (14 a) from the propylene oxide production (13) or from the ethylene oxide production (14) are fed to a polyether production (15) and the polyether (15 a) obtained therefrom is used in the production process (16) for producing new polyurethane material.

10. Method according to any of Claims 1 to 9, **characterized in that** the gas mixture (19 b) of carbon dioxide, carbon monoxide and gaseous hydrocarbons and possibly hydrogen obtained in the refining (19) in step b) is fed to the incineration (2) in step c).

11. Method according to any of Claims 1 to 9, **characterized in that** the gas mixture (19 b) obtained in the refining (19) in step b) is separated in a gas separation (30) into the components: carbon dioxide (2b), carbon monoxide (7c) and hydrocarbons (30a) and the components (2b; 7c; 30a) are reused individually, in particular **in that** the carbon dioxide (2b) is fed to the purification (4) in step d).

12. Method according to Claim 11, **characterized in that** the carbon monoxide (7c) from the gas separation (30) is fed to the isocyanate production (10).

13. Method according to any of Claims 1 to 12, **characterized in that** part of the polyurethane material (18 a) is fed directly to the incineration (2) in step c).

14. Method according to any of Claims 1 to 13, **characterized in that** at least part of the mixture (23) of carbon dioxide, carbon monoxide and possibly hydrogen obtained in step e) from the electrolysis (5) is fed directly to the methanol synthesis (11).

15. Method according to any of Claims 1 to 14, **characterized in that** oxygen (5a) is formed during the electrolysis (5) of carbon dioxide (4a) in step e), which is at least partially fed to the incineration (2) in step c).

16. Method according to any of Claims 1 to 15, **characterized in that** part of the carbon dioxide (6a) obtained in the separation (6) in step f) is optionally fed into the input stream (4a) of the electrolysis (5) in step e) and/or to the optional methanol synthesis (11).

## Revendications

1. Procédé pour la valorisation de déchets (18a) contenant du matériau à base de polyuréthane pour la fabrication de matières premières chimiques pour la préparation d'isocyanates et de polyuréthanes par
a) pyrolyse (1) du matériau à base de polyuréthane (18a) à température augmentée, le cas échéant en présence d'un catalyseur, avec obtention le cas échéant de dioxyde de carbone (la), le cas échéant de monoxyde de carbone, le cas échéant d'hydrogène (1d), d'un mélange (1c) d'hydrocarbures aliphatiques et aromatiques de bas poids moléculaire et d'hydrocarbures contenant de l'azote et d'un résidu (1b) de composés carbonés de poids moléculaire plus élevé,
b) le cas échéant raffinage (19) du mélange (1c) obtenu dans l'étape a) d'hydrocarbures de bas poids moléculaire avec obtention d'un mélange (19a) d'hydrocarbures gazeux et liquides et d'un mélange (19b) de dioxyde de carbone et de monoxyde de carbone, d'hydrogène et d'autres composés hydrocarbonés gazeux et séparation du mélange obtenu (19b) dans une séparation gazeuse 30,
c) combustion (2) du résidu (1b) obtenu dans l'étape a) et le cas échéant d'autres déchets de matériau à base de polyuréthane (18a) à l'aide de gaz contenant de l'oxygène (3a), en particulier à l'aide d'oxygène pur, avec obtention d'un gaz contenant du dioxyde de carbone (2a),
d) purification (4) du dioxyde de carbone (1a) et (2a) obtenu à partir de l'étape c) et le cas échéant de l'étape a) des constituants secondaires, en particulier des oxydes d'azote, des composés soufrés, de la poussière, de l'eau, de l'oxygène et de HCl, au choix par adsorption, lavage gazeux ou traitement catalytique avec obtention d'un dioxyde de carbone purifié (4a),
e) électrolyse (5) du dioxyde de carbone purifié (4a) obtenu dans l'étape d), en particulier transformation électrochimique du dioxyde de carbone (4a) sur une électrode à diffusion gazeuse avec obtention d'un mélange (23) au moins de monoxyde de carbone, de dioxyde de carbone non transformé et le cas échéant d'hydrogène,
f) séparation (6) du dioxyde de carbone non transformé (6a) du mélange (23) obtenu dans l'étape e) avec obtention d'un mélange (6b) au moins de monoxyde de carbone et le cas échéant d'hydrogène et recyclage du dioxyde de carbone non transformé (6a) dans l'électrolyse (5),
g) séparation facultative (7) de l'hydrogène (7a) le cas échéant obtenu à partir du mélange (6b) obtenu dans l'étape f) de monoxyde de carbone et le cas échéant d'hydrogène,
h) transformation du monoxyde de carbone (7b) ou (6b) obtenu à partir de l'étape g) ou f) avec du chlore (9a) en phosgène dans un procédé (10) pour la préparation d'isocyanate (10a),
i) le cas échéant transformation (16) de l'isocyanate (10a) obtenu à partir de l'étape h) avec des polyéthers (15a) et le cas échéant en plus avec des polyesters en un matériau fini (16a) à base de polyuréthane.

2. Procédé selon la revendication 1, **caractérisé en ce que** l'oxygène (3a) pour la combustion (2) dans l'étape c) est obtenu à partir d'une électrolyse de l'eau (3) ou d'une électrolyse de CO₂ (5).

3. Procédé selon la revendication 2, **caractérisé en ce que** l'hydrogène (3b) formé lors de l'électrolyse de l'eau (3) est utilisé au choix lors du raffinage (19) et/ou lors d'une hydrogénation (8) de nitroaromatiques, les amines (8a) obtenues lors de l'hydrogénation (8) de nitroaromatiques étant utilisées dans la préparation (10) d'isocyanate.

4. Procédé selon l'une quelconque des revendications 1 à 3, **caractérisé en ce que** l'hydrogène (7a) séparé, le cas échéant obtenu dans l'étape g), est utilisé lors de l'hydrogénation (8) de nitroaromatiques.

5. Procédé selon l'une quelconque des revendications 1 à 4, **caractérisé en ce que** le matériau à base de polyuréthane (16a) est recyclé, après son utilisation (17), en matériau de déchet à base de polyuréthane (17a) et les déchets isolés à base de polyuréthane sont utilisés comme matériau de départ (18a) dans l'étape a).

6. Procédé selon l'une quelconque des revendications 1 à 5, **caractérisé en ce que** le mélange (6b) obtenu dans les étapes e) et f) de monoxyde de carbone, le cas échéant de dioxyde de carbone et le cas échéant d'hydrogène est introduit dans une synthèse de méthanol (11).

7. Procédé selon l'une quelconque des revendications 1 à 6, **caractérisé**
**en ce qu'**au moins des flux partiels du monoxyde de carbone (22) et/ou de l'hydrogène (21) provenant de la séparation de gaz (7) ou de l'électrolyse de l'eau (3) sont introduits dans une synthèse de méthanol (11).

8. Procédé selon l'une quelconque des revendications 6 et 7, **caractérisé en ce que** le méthanol (11a) de la synthèse de méthanol (11) est introduit dans un procédé (12) de méthanol en oléfine (MTO) et les alcènes obtenus à partir de celui-ci sont ensuite introduits dans un procédé (13) ou (14) pour la préparation d'oxydes d'alkylène, en particulier d'oxyde de propylène (13a) et/ou d'oxyde d'éthylène (14a).

9. Procédé selon la revendication 8, **caractérisé en ce que** l'oxyde de propylène (13a) et/ou l'oxyde d'éthylène (14a) provenant de la préparation d'oxyde de propylène (13) ou de la préparation d'oxyde d'éthylène (14) est/sont introduits dans une préparation de polyéther (15) et le polyéther (15a) obtenu à partir de celui-ci est utilisé dans un procédé de préparation (16) pour la préparation de nouveau matériau à base de polyuréthane.

10. Procédé selon l'une quelconque des revendications 1 à 9, **caractérisé en ce que** le mélange gazeux (19b), obtenu lors du raffinage (19) dans l'étape b), de dioxyde de carbone, de monoxyde de carbone et d'hydrocarbures gazeux et le cas échéant d'hydrogène, est introduit dans la combustion (2) dans l'étape c).

11. Procédé selon l'une quelconque des revendications 1 à 9, **caractérisé en ce que** le mélange gazeux (19b), obtenu lors du raffinage (19) dans l'étape b), est séparé dans une séparation de gaz (30) en les composants : dioxyde de carbone (2b), monoxyde de carbone (7c) et hydrocarbures (30a) et les composants (2b ; 7c ; 30a) sont utilisés ultérieurement et individuellement, en particulier **en ce que** le dioxyde de carbone (2b) est introduit dans la purification (4) dans l'étape d).

12. Procédé selon la revendication 11, **caractérisé en ce que** le monoxyde de carbone (7c) provenant de la séparation des gaz (30) est introduit dans la préparation d'isocyanate (10).

13. Procédé selon l'une quelconque des revendications 1 à 12, **caractérisé**
**en ce qu'**une partie du matériau à base de polyuréthane (18a) est introduite directement dans la combustion (2) dans l'étape c).

14. Procédé selon l'une quelconque des revendications 1 à 13, **caractérisé**
**en ce qu'**au moins une partie du mélange (23) obtenu dans l'étape e) à partir de l'électrolyse (5), constitué de dioxyde de carbone, de monoxyde de carbone et le cas échéant d'hydrogène, est introduite directement dans la synthèse de méthanol (11).

15. Procédé selon l'une quelconque des revendications 1 à 14, **caractérisé en ce que** de l'oxygène (5a) est formé lors de l'électrolyse (5) de dioxyde de carbone (4a) dans l'étape e) et est introduit au moins partiellement dans la combustion (2) dans l'étape c).

16. Procédé selon l'une quelconque des revendications 1 à 15, **caractérisé**
**en ce qu'**une partie du dioxyde de carbone (6a) obtenu lors de la séparation (6) dans l'étape f) est introduite au choix dans le flux de départ (4a) de l'électrolyse (5) dans l'étape e) et/ou dans la synthèse de méthanol (11) facultative.
